# EUROPEAN PATENT SPECIFICATION

(11) **EP 3 826 525 B1**
(45) Date of publication and mention of the grant of the patent: **21.10.2026**
(21) Application number: 19841186.0
(22) Date of filing: 25.07.2019
(51) Int. Cl.: A61B 1/00, G06V 10/82, G06V 20/20, G16H 50/20, G16H 30/40, G06F 18/2413, G06V 10/764, A61B 1/313

(54) **METHODS, SYSTEMS, AND COMPUTER READABLE MEDIA FOR GENERATING AND PROVIDING ARTIFICIAL INTELLIGENCE ASSISTED SURGICAL GUIDANCE**
VERFAHREN, SYSTEME UND COMPUTERLESBARES MEDIUM ZUR ERZEUGUNG UND BEREITSTELLUNG VON CHIRURGISCHER FÜHRUNG MIT UNTERSTÜTZUNG DURCH KÜNSTLICHE INTELLIGENZ
PROCÉDÉS, SYSTÈMES ET SUPPORTS LISIBLES PAR ORDINATEUR POUR GÉNÉRER ET FOURNIR UN GUIDAGE CHIRURGICAL ASSISTÉ PAR INTELLIGENCE ARTIFICIELLE

(30) Priority: 25.07.2018 US 201862703400 P
(43) Date of publication of application: 02.06.2021
(73) Proprietor: The Trustees of The University of Pennsylvania, Philadelphia, PA 19104 (US)
(72) Inventor: BUCH, Vivek Paresh, Philadelphia, PA 19146 (US); MADSEN, Peter John, Philadelphia, PA 19146 (US); SHI, Jianbo, Philadelphia, PA 19147 (US)
(74) Representative: Isarpatent
(86) International application number: PCT/US2019/043428
(87) International publication number: WO 2020/023740

(56) References cited:
- WO-A1-2018/058509
- US-A1- 2013 038 707
- US-A1- 2013 038 707
- US-A1- 2016 259 888
- US-A1- 2016 259 888
- US-A1- 2017 360 412
- US-B2- 9 754 371
- ALAM ISRAT S ET AL: "Emerging Intraoperative Imaging Modalities to Improve Surgical Precision", MOLECULAR IMAGING & BIOLOGY, ELSEVIER, BOSTON, vol. 20, no. 5, 18 June 2018 (2018-06-18), pages 705 - 715, XP036590964, ISSN: 1536-1632, [retrieved on 20180618], DOI: 10.1007/S11307-018-1227-6

## Description

### PRIORITY CLAIM

This application claims the benefit of U.S. Provisional Patent Application Serial No. 62/703,400, filed July 25, 2018.

### TECHNICAL FIELD

The subject matter described herein relates to providing surgical guidance. More particularly, the subject matter described herein relates to methods, systems, and computer readable media for generating and providing artificial intelligence assisted surgical guidance.

### BACKGROUND

Artificial Intelligence (Al) technology is transforming the way we live. Autonomous cars, touchless cleaning robots, and personal assistants, such as 'Alexa', 'Siri', 'Cortana', and 'Google' are ushering humanity into a new world order. Heavy investments in this technology by the most influential companies of the current century have led to exponential growth in this industry over the past two to three years. One of the primary launching pads for expanding Al technology came from the famous IBM super computer known as Watson. Watson has since been recruited in the medical field to help physicians create differential diagnostic algorithms. As medical knowledge and disease states increase in complexity, the ability for the human brain to accurately identify and diagnose patients becomes increasingly difficult. In surgery, however, Al systems have not yet been readily applied. This is primarily due to the fact that until recently, machine learning technology was not powerful enough to be utilized in the surgical realm. However, the advent of computer vision framed within deep neural networks (DNNs), has significantly enhanced the capability of Al systems.

Accordingly, there exist a need for methods, systems, and computer readable media for generating and providing Al-assisted surgical guidance.

From the state of the art some applications of Al systems to assist in the surgical environment are known.

The US 9 754 371 B2 discloses a method and apparatus that uses machine learning to identify and predict abnormal cell growth in relation with brain tumors. The method is based on the comparison of a first set of images, which contain normal and abnormal regions and it is used as training data for the Al system, and a second image taken at a later time, in which the abnormalities are identified and indicated using the trained Al system.

The US 2016/259888 A1 discloses a method and system for pattern recognition using Al from video images of anatomical regions taken during a surgical intervention, in particular non-tissue regions. The Al-aided identification of these regions can be used in real-time by a surgeon as assistance during surgery.

The review article "Emerging Intraoperative Imaging Modalities to Improve Surgical Precision" by Alam et al (Molecular Imaging and Biology, Elsevier, Boston, vol. 20, no. 5, 18 June 2018, pages 705-715, ISSN; 1536-1632, DOI: 10.1007/S11307-018-1227-6) provides an overview of the state of the art of intraoperative imaging up to 2018, where some aspects of the potential of deep learning as applied to the surgical environment are anticipated.

The US 2013/038707 A1 discloses a display system and computer-implemented method for assisting a surgeon during surgery by deploying an augmented reality display system. A pre-operative image is processed by a computer to identify the relevant anatomical structures of the patient. This information is overlayed during a surgical procedure onto a real time video image on an interface, thus providing the surgeon with augmented reality information.

### SUMMARY

A method for generating and providing artificial intelligence assisted surgical guidance having the features of claim 1 is presented. The method includes analyzing video images from surgical procedures captured using an overhead operating room camera or microscope and training a neural network to identify anatomical objects (i.e. tissue types and critical structures), surgical objects (i.e. instruments and items), and tissue manipulation (i.e. the precise interface of anatomical and surgical objects) in video images. Training the neural network to identify tissue manipulations includes training the neural network to identify changes in contour of specific tissue types. The method includes a process by which the data is further grouped by surgery type and the neural network is trained on patterns in chronological and/or positional relationships between anatomical objects, surgical objects, and tissue manipulation during the course of each procedure. The method subsequently includes receiving, by the neural network, a live feed of video images from the surgery. The method further includes classifying, by the neural network, anatomical objects, surgical objects, and tissue manipulation in the live feed of video images. The method further includes outputting, in real time, by audio and video-overlay means, tailored surgical guidance based on the classified anatomical objects, surgical objects, and tissue manipulations in the live feed of video images, wherein outputting surgical guidance includes outputting a warning when a change in contour for a tissue type identified in the live video feed nears a damage threshold for the tissue type.

The subject matter described herein can be implemented in software in combination with hardware and/or firmware. For example, the subject matter described herein can be implemented in software executed by a processor. In one exemplary implementation, the subject matter described herein can be implemented using a non-transitory computer readable medium having stored thereon computer executable instructions that when executed by the processor of a computer control the computer to perform steps. Exemplary computer readable media suitable for implementing the subject matter described herein include non-transitory computer-readable media, such as disk memory devices, chip memory devices, programmable logic devices, and application specific integrated circuits. In addition, a computer readable medium that implements the subject matter described herein may be located on a single device or computing platform or may be distributed across multiple devices or computing platforms.

### BRIEF DESCRIPTION OF THE DRAWINGS

The subject matter described herein will now be explained with reference to the accompanying drawings of which:
Figure 1A is a block diagram of a system for generating and providing artificial intelligence assisted surgical guidance;
Figure 1B is a diagram illustrating a hierarchy of functions performed by the surgical guidance generator illustrated in Figure 1A;
Figure 2 is a flow chart illustrating an exemplary process for generating and providing artificial intelligence assisted surgical guidance;
Figures 3A-3C are examples of: A) a training video image; B) a labeled image produced from the training image; and C) the labeled image overlaid on the training image;
Figure 4 is a diagram of the custom neural network processing pipeline for monitoring surgical video frames over time to segment and track anatomical objects, surgical instruments, and interfaces between different anatomical objects and between the instruments and the anatomical objects;
Figure 5 includes intersection over union (IoU) graphs illustrating classification accuracy of the architecture illustrated in Figure 4;
Figures 6A and 6B are computer screen captures of surgical video frames at different times illustrating automated segmentation of anatomical objects and surgical instruments using the architecture illustrated in Figure 4.

### DETAILED DESCRIPTION

Every moment of a surgical procedure is defined by a decision. Some of these decisions will be mundane, but others require expert judgement and can alter a surgery's course, its outcome, and ultimately a patient's life. When distilled to its core, mastery in surgery requires the precise execution of intricate motor tasks and an ability to expertly analyze the operative field to correctly make these important decisions. The former is a skillset that is driven by human control. The latter, however, is dependent upon pattern recognition. No amount of physical operative talent can overcome surgical experience because the experienced surgeon is better at recognizing patterns in their operative environment. However, even the most experienced surgeons face many difficult decisions during crucial surgical checkpoints. The subject matter described herein includes a technology that can assist surgeons in real-time to recognize objects in their operative environment, notify them of situations that may require additional caution prior to executing an action, and assist surgeons in crucial decision-making situations.

Though the human brain is arguably the most complex machine ever created, Artificial Intelligence technology is transforming the way we live. In recent years, advancements in machine learning have led to the development of deep neural networks (DNNs), which have been able to consistently outperform the human brain in computational speed and, most importantly, pattern recognition. Our technology, the Artificial Operative Assistant (AOA), applies these advancements to the field of surgery to enhance decision-making and usher in the artificial intelligence revolution in surgery. The AOA provides real-time automated feedback on the surgical field to augment a surgeon's decision making and has the potential to become essential to any surgical procedure, constituting a new gold standard. Current work in this field has focused mostly on developing augmented reality systems that can overlay registered pre-operative imaging on an operative field to guide surgery or using computer vision technology to improve robotic surgery. Our technology is fundamentally different and novel, because it has the potential to be seamlessly adaptable to any surgery and is not dependent upon registration of imaging (a process that can be fraught with its own complications and difficulties) or restricted to robot-assisted procedures.

As a product, we would aim to launch our platform, known as TAIRIS, through either a cloud based or physical server. The end user-interface will be known as the AOA. Cloud based access would entail video feed processing in the cloud (in a HIPPA compliant manner) and results returned to the OR either through a boom audiovisual monitor or to a worn heads-up display. The computing power involved may prohibit this so we will also be prepared to create a physical processing device (local DNN CPU, with daily updates/communication between itself and the mainframe TAIRIS server) that can be integrated into an individual or suite of ORs. Either of these methods would require an initial purchase from a hospital or surgical group to integrate the system and we would also collect subscription fees that allow users to obtain updated and improved network processing as TAIRIS is continuously trained by end-user feedback.

In its first iteration, our technology utilizes computer vision based DNNs to continuously monitor an operative field and provide feedback to a surgeon in real-time with the goal of enhancing surgical decision making. In all operations, a video feed of the operative field can be obtained with either an overhead operating room (OR) camera, microscope, or endoscope. Regardless of the video input, the feed will be analyzed on a trained, multilayer, convolutional deep neural network capable of identifying key anatomical objects, surgical objects, and tissue manipulation. This real-time feedback and output of the trained DNN is considered the Artificial Operative Assistant (AOA).

To begin building this system, we will perform video processing on readily available microscopic and endoscopic recordings of cranial and spinal neurosurgical cases. Off-line image segmentation and labeling will be performed to break down components of representative video frames into matrices containing pixel-by-pixel categorizations of select anatomical and surgical objects. For example, in a lumbar microdiscectomy operation the pathological process involves a herniated lumbar intervertebral disc that is causing nerve root impingement resulting in pain, numbness, paresthesia, and weakness. The operation is performed with a small incision and a microscope to uncover the impinged nerve root and remove herniated disc material. Very often the distinction between nerve root and herniated disc is not clear. A few key categories can be identified in the microscopic recording of the operation including bone, ligament, thecal sac, nerve root, and disc. Images from this procedure will be labeled with these defined structures and then will be fed into our DNN.

Once initialized, this network will be trained using hundreds of these de-identified surgical videos in which the same key structures have been segmented out. Over multiple iterations, the deep neural network will coalesce classifiers for each pixel designating the anatomical or surgical object class to which it belongs. These DNN classifiers will then be utilized on-line to predict the most likely object class for each pixel during real-time video. Once this classifier can be verified for accuracy, it can be implemented during novel video capture. This output, namely the identified key elements of the surgical field, can then be returned to the surgeon in audiovisual from on a monitor or, in the future, a heads-up augmented reality display in real-time. This will appear as segmented overlays over the specific anatomic structures and provide probability estimates for each item to demonstrate the AOA's level of certainty (or uncertainty).

This foundational information and feedback will provide guidance to the surgeon as questions or uncertainty arise in the case. In this example, the trained DNN will be able to identify with some weighted probability what elements of the operative field belong to the nerve root versus disc material class. This concept can be extrapolated to any surgery. We plan to use multiple types of surgery to train the network to improve generalizability of the output to many surgical scenarios. Also, general labeling (i.e. "bone", "muscle", "blood vessel", etc.) will help to keep the scope of the network broad and capable of being used in many different types of surgery and even novel future applications.

Classifiers will also be set up to include tissue manipulation thresholds. By using contour detection techniques, we can actively monitor manipulation of key anatomic objects. This could be utilized to help determine potentially risky maneuvers prior to execution. For example, by defining the amount of manipulation an identified object can tolerate, the AOA will be able to provide feedback to the surgeon if they have or are about to exceed a safe level of manipulation. The AOA will also be able to caution surgeons if they are approaching structures designated as "do not manipulate," such as spinal cord during intradural spine surgery or the posterior capsule of the lens in cataract surgery.

In our second iteration, chronological and positional relationships between anatomical objects, surgical objects, and tissue manipulation over the course of a procedure will be entrained by the network. Quantifying these relationships will enable real-time object tracking and feedback for surgeons regarding normal stepwise procedure flow (chronological relationships) and upcoming "hidden" objects (positional relationships). The surgical guidance generator will utilize the foundational information from the neural network output as described above. This data will also be post-processed in a novel hierarchical algorithm framework and combined with other input data such as pre-operative imaging, patient demographic and co-morbidity factors, surgical object cost data, or intraoperative vital signs. These post-processed outputs of the surgical guidance generator will be clinically tailored, surgery- and even surgeon-specific including information such as a surgical roadmap of suggested next steps, movement efficiency metrics, complication avoidance warnings, procedural stepwise cost estimates, and predictive analytics that can provide novel intraoperative indices predicting post-operative outcome.

Furthermore, development of a graphical user interface will enable intra-operative and post-operative feedback on the AOA output accuracy which can be fed back into the DNN and allow for continuous training and propagation of the DNN. Post-operatively, the surgeon can be fed images onto a mobile device or tablet from the previous surgery where the AOA had some level of uncertainty about an object. The surgeon would provide feedback about the object and about the AOA's output about the object. This information can be fed back into the DNN to improve its training. This process can also occur intraoperatively. To accomplish this, we plan to develop an intraoperative user interface based on voice recognition and special labeling probe. A surgeon would be able to interact with the AOA using voice activation to ask questions of the AOA (i.e. "How certain are you that this is the disc material?", "How safe is it to retract this part of the thecal sac?") or ask the AOA to label objects as the user sees fit (i.e. "Label this object as the carotid artery."). Intraoperative feedback from the surgeon can also be fed back into the network to improve its output.

In the future, we foresee this technology being used on a digital, augmented reality enhanced, optical zoom capable surgical loupe headset where the video feed and subsequent visual feedback could be captured by and returned to the surgical loupe headset. As surgical headset technology improves, this type of Al system could become part of the surgical headpiece worn by every surgeon. This wide-reaching application of real-time, automated decision-making augmentation, surgical roadmaps, complication avoidance warnings, procedural stepwise cost estimates, predictive analytics, and tailored surgical guidance using artificial intelligence for surgeons is entirely novel; and our innovative AOA could redefine the forefront of cutting-edge surgical care. This technology also has the potential to transform surgical training and medical education. Trainees would gain valuable knowledge about anatomy and the operative field that most often takes years to hone. Medical students would be able to observe surgeries with the output of the AOA overlaid to improve their learning of anatomy.

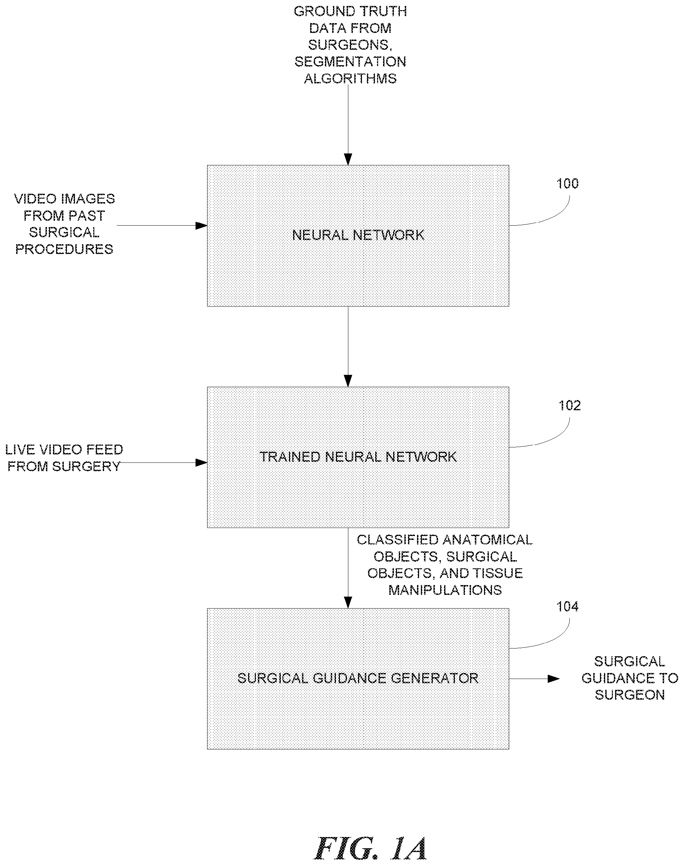

Figure 1A is a diagram illustrating exemplary systems for Al assisted surgical guidance. Referring to Figure 1A, a neural network 100 is trained from video images from past surgeries and ground truth data from surgeons to identify anatomical objects, surgical objects, and tissue manipulations in the video images. The result of the training is a trained neural network 102 that is trained to identify anatomical objects, surgical objects, and tissue manipulations.

Trained neural network 102 receives a live video feed from the surgery and outputs classifications of anatomical objects, surgical objects, and tissue manipulations. This information is output to a surgical guidance generator 104, which post-processes the neural network data through novel hierarchical algorithms to generate and output surgical guidance to a surgeon in real time during surgery. In the most basic example, the surgical guidance may be the anatomical object, surgical object, or tissue manipulation classifications superimposed on the regions of the images where these objects appear.

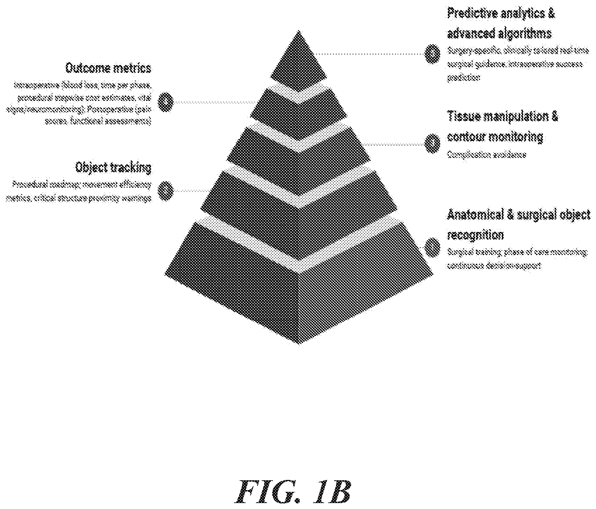

Figure 1B is a diagram illustrating a hierarchy of functions performed by the surgical guidance generator illustrated in Figure 1A. At the lowest level of the hierarchy, anatomical and surgical objects are identified. At the next level, movement of the anatomical and surgical objects is tracked. At this level critical structure proximity warnings along with procedural next-step suggestions may be generated. At the next level in the hierarchy, tissue manipulation and contour monitoring are performed to warn the surgeon about complications that may result from manipulating tissue beyond safe mechanical ranges. At the next level in the hierarchy, intraoperative outcome metrics may be generated and utilized to create feedback to the surgeon. Examples of intraoperative outputs that may be generated include blood loss, time per phase, procedural stepwise cost estimates, vital signs, and neuromonitoring data. Similarly, postoperative metrics may also be correlated and utilized to create feedback to the surgeon. These postoperative metrics may include pain scores and functional assessments. At the next level in the hierarchy, predictive analytics and advanced algorithms may be used to generate and output surgery specific, clinically tailored real time surgical guidance and an intraoperative prediction of success of a surgical procedure. Specific examples of output that may be generated by the system illustrated in Figure 1A will now be described.

In one example, neural network 100 may be trained to identify bone, muscle, tendons, organs, blood vessels, nerve roots, as well as abnormal tissues, including tumor.

According to the invention, neural network 100 is trained to identify changes in contour of specific tissue types. In such a case, surgical guidance generator 104 outputs a warning when a change in contour for tissue type identified in the live video feed nears a damaged threshold for the tissue type. Such output may prevent the surgeon from damaging tissue during the surgery.

In another example, neural network 100 may be trained to identify and track changes in anatomical objects, surgical objects, and tissue manipulations over the course of surgical procedures and over time.

In another example, neural network 100 may be trained to identify a pointer instrument having a pointer end that when brought in close proximity to a tissue type in the live video feed triggers surgical guidance generator 104 to generate output identifying the tissue type. For example, when the pointer is brought in close proximity to bone, surgical guidance generator 104 may generate output indicating the word "bone" superimposed on the bone tissue in the live video feed, or as an auditory signal saying the word "bone", during the surgery. When using surgery-specific anatomical object outputs, "bone" could be replaced by a more specific anatomical structure seen in the given operation, such as "lamina" or "facet joint" during spine surgery.

In another example, algorithms that process and display output from neural network 102 in surgery and surgeon-specific manners may be used in combination with trained neural network 102 to provide enhanced surgical guidance. This can include a surgical roadmap with suggested next steps at each time point based on chronologically processed data for each surgery type, or a continuous extent-of-resection calculation for tumor resection surgery by utilizing volumetric analysis of the resection cavity in real-time. Surgeon-specific metrics could include movement efficiency metrics based on, for example, the amount of time a particular instrument was used for a given step in the procedure. The surgeon could use this type of active feedback over time to improve their own operative efficiency. Cost information of various surgical objects could also be incorporated to enable active feedback of cost during each step to reduce behaviors leading to surgical waste.

In yet another example, surgical guidance generator 104 may process data from multiple input sources, including pre-operative imaging, patient-specific risk factors, and intraoperative vital signs and output such data superimposed on the live video feed of the surgery to provide surgical guidance to the surgeon.

Surgical guidance generator 104, in one example, may output the surgical guidance on a video screen that surgeons use during image guided surgery. The video screen may be standalone display separate from the surgical field or an augmented reality display located in or in front of the surgical field.

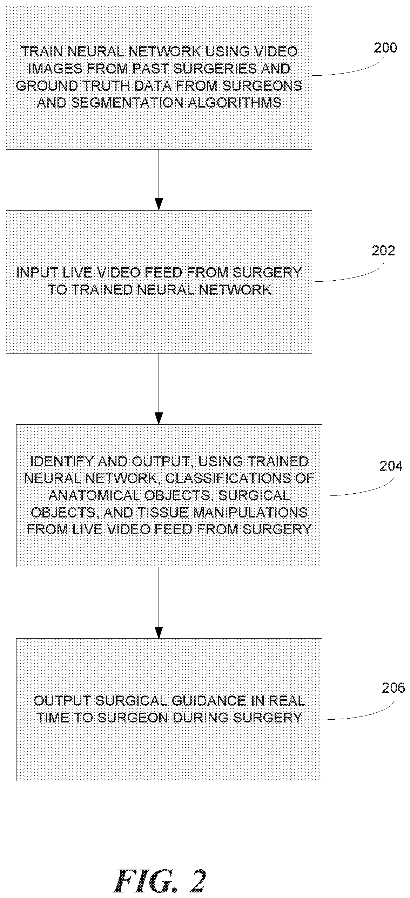

Figure 2 is a flow chart illustrating an exemplary process or providing artificial intelligence assisted surgical guidance. Referring to Figure 2, in step 200, video images from surgical procedures are analyzed and used to train a neural network to identify anatomical objects, surgical objects, and tissue manipulations in the video images. For example, neural network 100 may be trained using video images from past surgeries and ground truth data from surgeons and/or segmentation algorithms that identify anatomical objects, surgical objects, and tissue manipulations in the video images. Training the neural network to identify tissue manipulations includes training the neural network to identify changes in contour of specific tissue types.

In step **202,** the neural network receives a live feed of video images from a surgery in progress. For example, trained neural network **102** may be provided with live video feed from a surgery.

In step **204,** trained neural network **102** identifies anatomical objects, surgical objects, and tissue manipulations from the live video feed from the surgery and outputs classifications of the anatomical objects, surgical objects, and tissue manipulations. For example, trained neural network **102** may output the classifications to surgical guidance generator **104** illustrated in Figure 1A.

In step **206,** surgical guidance is generated based on the classified anatomical objects, surgical objects, and tissue manipulations in the live video feed. For example, surgical guidance generator **104** may output surgical guidance to surgeons based on anatomical objects, surgical objects, and tissue manipulations classified by trained neural network **102.** In addition, the surgical guidance may include data from other sources, such as pre-operative images or other patient or surgery specific data. Outputting surgical guidance includes outputting a warning when a change in contour for a tissue type identified in the live video feed nears a damage threshold for the tissue type.

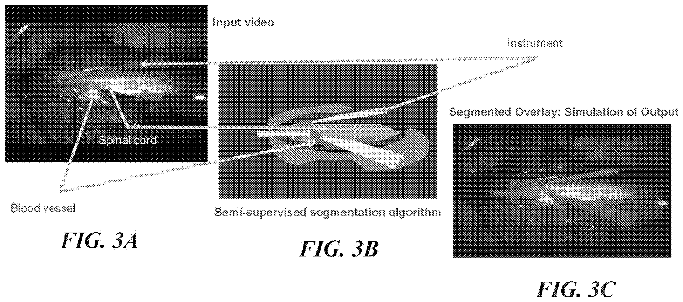

Figures 3A through 3C illustrate exemplary training of neural network **100.** More particularly, Figure 3A illustrates an image of an abnormal blood vessel around the spinal cord from a video taken during a surgery. Figure 3B illustrates segmentation of the image in Figure 3A identifying a surgical instrument, the spinal cord, and the blood vessels. Figure 3C illustrates an overlay of the segmentation and the image of the blood vessel. The segmentation data in Figure 3B may be used to train neural network **100** to identify the anatomical objects (the blood vessel and the spinal cord) and the surgical instrument. Once trained, the neural network can output segmented overlays, such as that illustrated in Figure 3C.

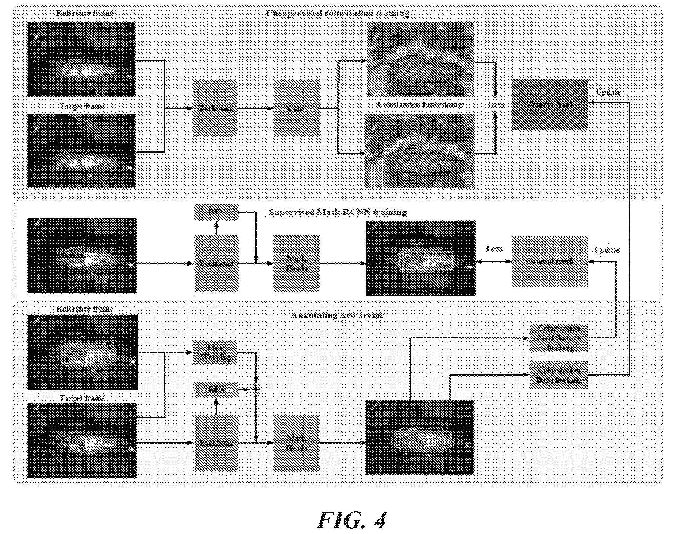

Figure 4 is a diagram of the custom neural network processing pipeline for monitoring surgical video frames over time to segment and track anatomical objects, surgical instruments, and interfaces between different anatomical objects and between the instruments and the anatomical objects. In Figure 4 the topmost rectangle labeled unsupervised colorization training illustrates unsupervised training of a convolutional neural network to identify object contours based on color properties of the images. The inputs to convolutional neural network are a target frame and a reference frame, where the reference frame is a frame depicting a surgical field at a first time point and the target frame is a frame depicting the surgical field at a second time point after the first time. The boxes labeled Backbone and Conv represent the convolutional neural network being trained. The convolutional neural network backbone may be modified from the Detectron mask recurrent convolutional neural network (mask RCNN) made available by Facebook at https://github.com/facebookresearch/Detectron or other publicly available Python-based CNNs.

Continuing with the unsupervised training rectangle in Figure 4, the outputs of the neural network are colorizations of the reference frame and the target frame, where likely object contours are defined in terms of color data. The loss represents the difference in colorization between the colorization produced from the reference frame and the colorization produced from the target frame. Over numerous video frames this unsupervised algorithm creates a memory bank of sequential colorization embeddings which can then be used to inform the outputs of the trained custom neural network

The central rectangle in Figure 4 represents supervised training of the mask RCNN using ground truth image data labeled by surgical experts. Because there can be thousands of image frames in every surgery and manual labeling of surgical images is time consuming and requires a surgical expert, it is desirable to only manually label a subset of images from a surgery to train the RCNN. In the supervised training of the RCNN, the RCNN receives as input an image to be classified and ground truth data including one or more labeled surgical images frames. A region proposal network (RPN) receives image data from the backbone and outputs proposed regions of interest to inform object segmentation. The mask heads create proposed labels for anatomical objects in the image to be classified. The RCNN produces a surgical image with instance segmentations around classified anatomical structures and surgical instruments. The RCNN also outputs a loss indicative of a difference between the network-classified and manually classified image.

The lowermost rectangle in Figure 4 represents the semi-supervised annotation of a target frame with unknown anatomical objects and/or surgical instruments using the sparsely trained RCNN. In the lowermost rectangle, the RCNN receives as input the target frame to be classified. A flow warping algorithm calculates the movement trajectory of each segmented object from the preceding frames to guide the RPN in choosing regions of interest. The mask RCNN segments the target frame. The output is checked against the memory bank to refine network outputs. This is in turn used to update the suggested pixel-wise object contours and bounding box contours stored in the memory bank. Thus, the system illustrated in Figure 4 learns as it classifies each new image. This process enables a methodology to minimize manual labeling of surgical data while still enabling high classification accuracy of the custom neural network outputs.

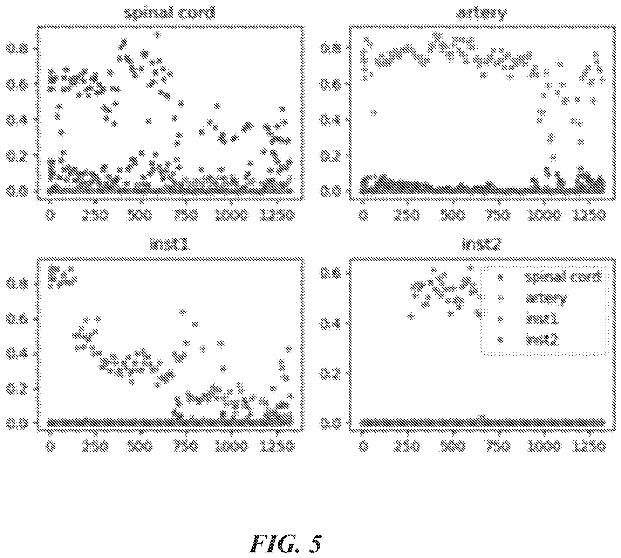

Figure 5 includes intersection over union (IoU) graphs illustrating classification accuracy of the architecture illustrated in Figure 4. In Figure 5, the horizontal axis of each graph represents different frames over time. The vertical axis represents intersection over union, which is a measure of percentage accuracy of the classification. The upper left graph in Figure 5 shows classification accuracy of the spinal cord clustered mainly between 60% and 80%. The upper right graph in Figure 5 shows similar accuracy for classification of an artery. The lower graphs illustrate classification accuracy for a first instrument, labeled instr1 that begins about 80% and decreases over time. The classification of inst2 zero, due to the co-classification of instrument 1 and instrument 2.

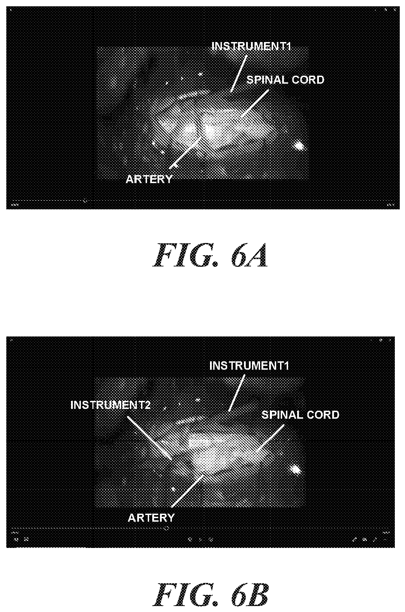

Figures 6A and 6B are computer screen shots of surgical video frames at different times illustrating segmentation of anatomical objects and instruments using the architecture illustrated in Figure 4. The textual labels in Figures 6A and 6B were added manually for illustrative purposes but could have been added by the network illustrated in Figure 4. In Figure 6A, the video frame shows an artery, the spinal cord, and an instrument, labeled INSTRUMENT1. The artery, spinal cord and INSTRUMENT1 are segmented by the network illustrated in Figure 4, and that segmentation is depicted in the video frames as transparent color of different colors on top of each anatomical object or image. For example, the artery may be overlaid with a blue transparent overlay, the spinal cord may be overlaid with a yellow transparent overlay, and the instrument may be overlaid with a purple transparent overlay. Figure 6B illustrates a subsequent video frame and how the segmentation performed by the network illustrated in Figure 4 tracks movement of the anatomical objects and instruments. In Figure 6B, the artery has been moved to a new position by a second instrument, labeled INSTRUMENT2. Thus, the trained RCNN illustrated in Figure 4 segments anatomical objects and surgical instruments in a live video feed from surgery and tracks movements of the instruments and anatomical objects over time.

It will be understood that various details of the presently disclosed subject matter may be changed without departing from the scope of the presently disclosed subject matter. Furthermore, the foregoing description is for the purpose of illustration only, and not for the purpose of limitation.

## Claims

1. A processor-implemented method for generating and providing artificial intelligence assisted surgical guidance, the method comprising:
analyzing (200) video images from surgical procedures captured using an overhead operating room camera or microscope and training a neural network (100) to identify anatomical objects, surgical objects, and tissue manipulations in the video images;
receiving (202), by the trained neural network (102), a live feed of video images from a surgery;
classifying (204), by the trained neural network (102), anatomical objects, surgical objects, and tissue manipulations in the live feed of video images; and
outputting (206), in real time, surgical guidance based on the classified anatomical objects, surgical objects, and tissue manipulations in the live feed of video images, wherein training the neural network (100) to identify tissue manipulations includes training the neural network (100) to identify changes in contour of specific tissue types and wherein outputting surgical guidance includes outputting a warning when a change in contour for a tissue type identified in the live video feed nears a damage threshold for the tissue type.

2. The method of claim 1, wherein training the neural network (100) to identify anatomical objects includes training the neural network (100) to identify bone, muscle, tendons, organs, blood vessels, nerve roots, as well as abnormal tissues including tumor.

3. The method of claim 1, wherein training the neural network (100) includes training the neural network (100) to identify and track changes in the anatomical objects, surgical objects, and tissue manipulations over the course of each of the surgical procedures and over time.

4. The method of claim 1, wherein training the neural network (100) to identify surgical objects includes training the neural network (100) to identify a pointer instrument having a pointer end that when brought in close proximity to an anatomical object in a live feed of video from a surgery triggers generating of output identifying the anatomical object.

5. The method of claim 1, wherein outputting (206) the surgical guidance includes developing algorithms that further process and display output from the trained neural network (102) in surgeon- and surgery-specific manners including movement efficiency metrics and intraoperative metrics to predict success of each surgery.

6. The method of claim 1, wherein the neural network (100) comprises a mask recurrent convolutional neural network and wherein training the neural network (100) includes performing semi-supervised training of the mask recurrent neural network to detect patterns of the anatomical objects, surgical objects and tissue manipulations in the video frames in combination with supervised training of the mask recurrent convolutional neural network using labeled surgical image frames.

7. A system for generating and providing artificial intelligence assisted surgical guidance, the system comprising:
at least one processor;
a neural network (100) implemented by the at least one processor and trained through analysis of video images from surgical procedures to identify anatomical objects, surgical objects, and tissue manipulations in the video images;
the trained neural network (102) being configured to receive, a live feed of video images from a surgery and classify anatomical objects, surgical objects, and tissue manipulations in the live feed of video images; and
a surgical guidance generator (104) for outputting, in real time, guidance based on the classified anatomical objects, surgical objects, and tissue manipulations in the live feed of video images, wherein the neural network (100) is trained to identify changes in contour of specific tissue types and wherein the surgical guidance generator (104) is configured to output a warning when a change in contour for a tissue type identified in the live video feed nears a damage threshold for the tissue type.

8. The system of claim 7, wherein the neural network (100) is trained to identify tissue including bone, muscle, tendons, organs, blood vessels, and nerve roots, as well as abnormal tissues including tumor.

9. The system of claim 7, wherein the neural network (100) is trained to identify and track changes over time and across multiple surgical procedures in anatomical objects, surgical objects, and tissue manipulations.

10. The system of claim 7, wherein the neural network (100) is trained to identify a pointer instrument having a pointer end that when brought in close proximity to an anatomical object in a live feed of video from a surgery triggers the surgical guidance generator to generate output identifying the anatomical object.

11. The system of claim 7, wherein outputting the surgical guidance includes further processing and displaying output of the trained neural network (102) in surgeon- and surgery-specific manners including movement efficiency metrics and intraoperative metrics predicting success of each surgery.

12. The system of claim 7, wherein the neural network (100) comprises a mask recurrent convolutional neural network trained by performing semi-supervised training of the mask recurrent neural network to detect patterns of the anatomical objects, surgical objects and tissue manipulations in the video frames in combination with supervised training of the mask recurrent convolutional neural network using labeled surgical image frames.

13. A non-transitory computer readable medium having stored thereon executable instructions that when executed by a processor of a computer control the computer to perform steps comprising:
analyzing (200) video images from surgical procedures and training a neural network (100) to identify anatomical objects, surgical objects, and tissue manipulations in the video images;
receiving (202), by the trained neural network (102), a live feed of video images from a surgery;
classifying (204), by the trained neural network (102), anatomical objects, surgical objects, and tissue manipulations in the live feed of video images; and
outputting (206), in real time, surgical guidance based on the classified anatomical objects, surgical objects, and tissue manipulations in the live feed of video images, wherein training the neural network (100) to identify tissue manipulations includes training the neural network (100) to identify changes in contour of specific tissue types and wherein outputting surgical guidance includes outputting a warning when a change in contour for a tissue type identified in the live video feed nears a damage threshold for the tissue type.

## Patentansprüche

1. Prozessorimplementiertes Verfahren zum Generieren und Bereitstellen einer durch künstliche Intelligenz unterstützten chirurgischen Führung, wobei das Verfahren umfasst:
Analysieren (200) von Videobildern von chirurgischen Eingriffen, die unter Verwendung einer Überkopf-Operationssaalkamera oder eines Mikroskops aufgenommen wurden, und Trainieren eines neuronalen Netzwerks (100) auf das Identifizieren von anatomischen Objekten, chirurgischen Objekten und Gewebemanipulationen in den Videobildern;
Empfangen (202), durch das trainierte neuronale Netzwerk (102), eines Live-Feeds von Videobildern von einem chirurgischen Eingriff;
Klassifizieren (204), durch das trainierte neuronale Netzwerk (102), von anatomischen Objekten, chirurgischen Objekten und Gewebemanipulationen in dem Live-Feed von Videobildern; und
Ausgeben (206), in Echtzeit, einer chirurgischen Führung auf der Grundlage der klassifizierten anatomischen Objekte, chirurgischen Objekte und Gewebemanipulationen in dem Live-Feed von Videobildern, wobei das Trainieren des neuronalen Netzwerks (100) auf das Identifizieren von Gewebemanipulationen das Trainieren des neuronalen Netzwerks (100) auf das Identifizieren von Veränderungen einer Kontur spezieller Gewebetypen enthält, und wobei das Ausgeben einer chirurgischen Führung das Ausgeben einer Warnung enthält, wenn sich eine Veränderung einer Kontur für einen in dem Live-Video-Feed identifizierten Gewebetyp einer Schadensschwelle für den Gewebetyp nähert.

2. Verfahren nach Anspruch 1, wobei das Trainieren des neuronalen Netzwerks (100) auf das Identifizieren von anatomischen Objekten das Trainieren des neuronalen Netzwerks (100) auf das Identifizieren von Knochen, Muskeln, Sehnen, Organen, Blutgefäßen, Nervenwurzeln sowie von abnormalen Geweben einschließlich Tumoren enthält.

3. Verfahren nach Anspruch 1, wobei das Trainieren des neuronalen Netzwerks (100) das Trainieren des neuronalen Netzwerks (100) auf das Identifizieren und Nachverfolgen von Veränderungen in den anatomischen Objekten, chirurgischen Objekten und Gewebemanipulationen im Verlauf eines jeden der chirurgischen Eingriffe und im zeitlichen Verlauf enthält.

4. Verfahren nach Anspruch 1, wobei das Trainieren des neuronalen Netzwerks (100) auf das Identifizieren von chirurgischen Objekten das Trainieren des neuronalen Netzwerks (100) auf das Identifizieren eines Zeigerinstruments enthält, das ein Zeigerende aufweist, das, wenn es in unmittelbare Nähe zu einem anatomischen Objekt in einem Live-Feed eines Videos von einem chirurgischen Eingriff gebracht wird, das Generieren einer Ausgabe auslöst, die das anatomische Objekt identifiziert.

5. Verfahren nach Anspruch 1, wobei das Ausgeben (206) der chirurgischen Führung das Entwickeln von Algorithmen enthält, die die Ausgabe von dem trainierten neuronalen Netzwerk (102) auf Chirurgen- und Chirurgie-spezifische Weisen weiterverarbeiten und anzeigen, einschließlich Bewegungseffizienzkennzahlen und intraoperativer Kennzahlen, um den Erfolg jedes chirurgischen Eingriffs zu prognostizieren.

6. Verfahren nach Anspruch 1, wobei das neuronale Netzwerk (100) ein rekurrentes neuronales Masken-Faltungsnetzwerk umfasst, und wobei das Trainieren des neuronalen Netzwerks (100) das Durchführen eines halb-beaufsichtigten Trainierens des rekurrenten neuronalen Masken-Netzwerks auf das Detektieren von Mustern der anatomischen Objekte, chirurgischen Objekte und Gewebemanipulationen in den Video-Frames in Kombination mit einem beaufsichtigten Trainieren des rekurrenten neuronalen Masken-Faltungsnetzwerks unter Verwendung markierter chirurgischer Bild-Frames provenant.

7. System zum Generieren und Bereitstellen einer durch künstliche Intelligenz unterstützten chirurgischen Führung, wobei das System umfasst:
mindestens einen Prozessor;
ein neuronales Netzwerk (100), das durch den mindestens einen Prozessor implementiert wird und durch Analyse von Videobildern von chirurgischen Eingriffen auf das Identifizieren von anatomischen Objekten, chirurgischen Objekten und Gewebemanipulationen in den Videobildern trainiert wurde;
wobei das trainierte neuronale Netzwerk (102) dazu ausgebildet ist, einen Live-Feed von Videobildern von einem chirurgischen Eingriff zu empfangen und anatomische Objekte, chirurgische Objekte und Gewebemanipulationen in dem Live-Feed von Videobildern zu klassifizieren; und
einen Generator (104) für chirurgische Führung zum Ausgeben, in Echtzeit, einer Führung auf der Grundlage der klassifizierten anatomischen Objekte, chirurgischen Objekte und Gewebemanipulationen in dem Live-Feed von Videobildern, wobei das neuronale Netzwerk (100) auf das Identifizieren von Veränderungen einer Kontur spezieller Gewebetypen trainiert ist, und wobei der Generator (104) für chirurgische Führung dazu ausgebildet ist, eine Warnung auszugeben, wenn sich eine Veränderung einer Kontur für einen in dem Live-Video-Feed identifizierten Gewebetyp einer Schadensschwelle für den Gewebetyp nähert.

8. System nach Anspruch 7, wobei das neuronale Netzwerk (100) auf das Identifizieren von Gewebe einschließlich Knochen, Muskeln, Sehnen, Organen, Blutgefäßen und Nervenwurzeln sowie abnormalen Geweben einschließlich Tumoren trainiert ist.

9. System nach Anspruch 7, wobei das neuronale Netzwerk (100) auf das Identifizieren und Nachverfolgen von Veränderungen in anatomischen Objekten, chirurgischen Objekten und Gewebemanipulationen im zeitlichen Verlauf und über mehrere chirurgischen Eingriffe hinweg trainiert ist.

10. System nach Anspruch 7, wobei das neuronale Netzwerk (100) auf das Identifizieren eines Zeigerinstruments trainiert ist, das ein Zeigerende aufweist, das, wenn es in unmittelbare Nähe zu einem anatomischen Objekt in einem Live-Feed eines Videos von einem chirurgischen Eingriff gebracht wird, den Generator für chirurgische Führung auslöst, eine Ausgabe zu generieren, die das anatomische Objekt identifiziert.

11. System nach Anspruch 7, wobei das Ausgeben der chirurgischen Führung das Weiterverarbeiten und Anzeigen der Ausgabe des trainierten neuronalen Netzwerks (102) auf Chirurgen- und Chirurgie-spezifische Weisen enthält, einschließlich Bewegungseffizienzkennzahlen und intraoperativer Kennzahlen, die den Erfolg jedes chirurgischen Eingriffs prognostizieren.

12. System nach Anspruch 7, wobei das neuronale Netzwerk (100) ein rekurrentes neuronales Masken-Faltungsnetzwerk umfasst, das mittels Durchführen eines halb-beaufsichtigten Trainierens des rekurrenten neuronalen Masken-Netzwerks auf das Detektieren von Mustern der anatomischen Objekte, chirurgischen Objekte und Gewebemanipulationen in den Video-Frames in Kombination mit einem beaufsichtigten Trainieren des rekurrenten neuronalen Masken-Faltungsnetzwerks unter Verwendung markierter chirurgischer Bild-Frames trainiert wird.

13. Nicht-transitorisches computerlesbares Medium, auf dem ausführbare Instruktionen gespeichert sind, die, wenn sie durch einen Prozessor eines Computers ausgeführt werden, den Computer steuern, Schritte auszuführen, die umfassen:
Analysieren (200) von Videobildern von chirurgischen Eingriffen und Trainieren eines neuronalen Netzwerks (100) auf das Identifizieren anatomischer Objekte, chirurgischer Objekte und Gewebemanipulationen in den Videobildern;
Empfangen (202), durch das trainierte neuronale Netzwerk (102), eines Live-Feeds von Videobildern von einem chirurgischen Eingriff;
Klassifizieren (204), durch das trainierte neuronale Netzwerk (102), von anatomischen Objekten, chirurgischen Objekten und Gewebemanipulationen in dem Live-Feed von Videobildern; und
Ausgeben (206), in Echtzeit, einer chirurgischen Führung auf der Grundlage der klassifizierten anatomischen Objekte, chirurgischen Objekte und Gewebemanipulationen in dem Live-Feed von Videobildern, wobei das Trainieren des neuronalen Netzwerks (100) auf das Identifizieren von Gewebemanipulationen das Trainieren des neuronalen Netzwerks (100) auf das Identifizieren von Veränderungen einer Kontur spezieller Gewebetypen umfasst, und wobei das Ausgeben einer chirurgischen Führung das Ausgeben einer Warnung umfasst, wenn sich eine Veränderung einer Kontur für einen in dem Live-Video-Feed identifizierten Gewebetyp einer Schadensschwelle für den Gewebetyp nähert.

## Revendications

1. Procédé mis en œuvre par un processeur pour générer et fournir un guidage chirurgical assisté par intelligence artificielle, le procédé comprenant les étapes consistant à :
analyser (200) des images vidéo provenant de procédures chirurgicales capturées à l'aide d'une caméra installée au plafond d'une salle d'opération ou d'un microscope et entraîner un réseau neuronal (100) afin d'identifier des objets anatomiques, des objets chirurgicaux et des manipulations tissulaires dans les images vidéo ;
recevoir (202), par le réseau neuronal entraîné (102), un flux en direct d'images vidéo provenant d'une opération chirurgicale ;
classer (204), par le réseau neuronal entraîné (102), les objets anatomiques, les objets chirurgicaux et les manipulations tissulaires dans le flux en direct d'images vidéo ; et
produire (206), en temps réel, un guidage chirurgical sur la base des objets anatomiques, des objets chirurgicaux et des manipulations tissulaires classés dans le flux en direct d'images vidéo, dans lequel l'entraînement du réseau neuronal (100) à identifier des manipulations tissulaires inclut l'entraînement du réseau neuronal (100) à identifier des changements dans le contour de types de tissus spécifiques, et dans lequel la production d'un guidage chirurgical inclut l'émission d'un avertissement lorsqu'un changement de contour pour un type de tissu identifié dans le flux vidéo en direct s'approche d'un seuil de lésion pour le type de tissu.

2. Procédé selon la revendication 1, dans lequel l'entraînement du réseau neuronal (100) à identifier des objets anatomiques inclut l'entraînement du réseau neuronal (100) à identifier des os, des muscles, des tendons, des organes, des vaisseaux sanguins, des racines nerveuses ainsi que des tissus anormaux, y compris des tumeurs.

3. Procédé selon la revendication 1, dans lequel l'entraînement du réseau neuronal (100) inclut l'entraînement du réseau neuronal (100) à identifier et à suivre des changements dans les objets anatomiques, les objets chirurgicaux et les manipulations tissulaires au cours de chacune des procédures chirurgicales et au cours du temps.

4. Procédé selon la revendication 1, dans lequel l'entraînement du réseau neuronal (100) à identifier des objets chirurgicaux inclut l'entraînement du réseau neuronal (100) à identifier un instrument de pointage comportant une extrémité de pointage qui, lorsqu'elle est amenée à proximité immédiate d'un objet anatomique dans un flux vidéo en direct provenant d'une opération chirurgicale, déclenche la génération d'une sortie identifiant l'objet anatomique.

5. Procédé selon la revendication 1, dans lequel la production (206) du guidage chirurgical inclut le développement d'algorithmes qui traitent et affichent de manière plus approfondie la sortie du réseau neuronal entraîné (102) selon des modalités spécifiques au chirurgien et à l'opération chirurgicale, y compris des mesures d'efficacité de mouvements et des mesures peropératoires permettant de prédire la réussite de chaque opération chirurgicale.

6. Procédé selon la revendication 1, dans lequel le réseau neuronal (100) comprend un réseau neuronal convolutif récurrent à masque et dans lequel l'entraînement du réseau neuronal (100) inclut l'exécution d'un entraînement semi-supervisé du réseau neuronal récurrent à masque pour détecter des motifs des objets anatomiques, des objets chirurgicaux et des manipulations tissulaires dans les images vidéo, en combinaison avec un entraînement supervisé du réseau neuronal convolutif récurrent à masque à l'aide d'images chirurgicales étiquetées.

7. Système destiné à générer et à fournir un guidage chirurgical assisté par intelligence artificielle, le système comprenant :
au moins un processeur ;
un réseau neuronal (100) mis en œuvre par ledit au moins un processeur et entraîné par l'analyse d'images vidéo provenant de procédures chirurgicales afin d'identifier des objets anatomiques, des objets chirurgicaux et des manipulations tissulaires dans les images vidéo ;
le réseau neuronal entraîné (102) étant configuré pour recevoir un flux en direct d'images vidéo d'une opération chirurgicale et pour classer des objets anatomiques, des objets chirurgicaux et des manipulations tissulaires dans le flux en direct d'images vidéo ; et
un générateur de guidage chirurgical (104) destiné à produire, en temps réel, un guidage sur la base des objets anatomiques, des objets chirurgicaux et des manipulations tissulaires classés dans le flux en direct d'images vidéo, dans lequel le réseau neuronal (100) est entraîné à identifier des changements dans le contour de types de tissus spécifiques et dans lequel le générateur de guidage chirurgical (104) est configuré pour émettre un avertissement lorsqu'un changement de contour d'un type de tissu identifié dans le flux vidéo en direct s'approche d'un seuil de lésion pour ce type de tissu.

8. Système selon la revendication 7, dans lequel le réseau neuronal (100) est entraîné à identifier des tissus incluant des os, des muscles, des tendons, des organes, des vaisseaux sanguins et des racines nerveuses ainsi que des tissus anormaux, y compris des tumeurs.

9. Système selon la revendication 7, dans lequel le réseau neuronal (100) est entraîné à identifier et à suivre des changements au cours du temps et sur plusieurs procédures chirurgicales concernant des objets anatomiques, des objets chirurgicaux et des manipulations tissulaires.

10. Système selon la revendication 7, dans lequel le réseau neuronal (100) est entraîné à identifier un instrument de pointage comportant une extrémité de pointage qui, lorsqu'elle est amenée à proximité immédiate d'un objet anatomique dans un flux vidéo en direct provenant d'une opération chirurgicale, déclenche le générateur de guidage chirurgical afin qu'il génère une sortie identifiant l'objet anatomique.

11. Système selon la revendication 7, dans lequel la production du guidage chirurgical inclut un traitement et un affichage plus approfondis de la sortie du réseau neuronal entraîné (102) selon des modalités spécifiques au chirurgien et à l'opération chirurgicale, y compris des mesures d'efficacité de mouvements et des mesures peropératoires permettant de prédire la réussite de chaque opération chirurgicale.

12. Système selon la revendication 7, dans lequel le réseau neuronal (100) comprend un réseau neuronal convolutif récurrent à masque entraîné en effectuant un entraînement semi-supervisé du réseau neuronal récurrent à masque pour détecter des motifs des objets anatomiques, des objets chirurgicaux et des manipulations tissulaires dans les images vidéo, en combinaison avec un entraînement supervisé du réseau neuronal convolutif récurrent à masque à l'aide d'images chirurgicales étiquetées.

13. Support non transitoire lisible par ordinateur sur lequel sont stockées des instructions exécutables qui, lorsqu'elles sont exécutées par un processeur d'un ordinateur, commandent à celui-ci d'effectuer les étapes consistant à :
analyser (200) des images vidéo provenant de procédures chirurgicales et entraîner un réseau neuronal (100) à identifier des objets anatomiques, des objets chirurgicaux et des manipulations tissulaires dans les images vidéo ;
recevoir (202), par le réseau neuronal entraîné (102), un flux en direct d'images vidéo provenant d'une opération chirurgicale ;
classer (204), par le réseau neuronal entraîné (102), les objets anatomiques, les objets chirurgicaux et les manipulations tissulaires dans le flux en direct d'images vidéo ; et
produire (206), en temps réel, un guidage chirurgical sur la base des objets anatomiques, des objets chirurgicaux et des manipulations tissulaires classés dans le flux en direct d'images vidéo, dans lequel l'entraînement du réseau neuronal (100) à identifier des manipulations tissulaires inclut l'entraînement du réseau neuronal (100) à identifier des changements dans le contour de types de tissus spécifiques, et dans lequel la production d'un guidage chirurgical inclut l'émission d'un avertissement lorsqu'un changement de contour pour un type de tissu identifié dans le flux vidéo en direct s'approche d'un seuil de lésion pour ce type de tissu.
